# EUROPEAN PATENT APPLICATION

(11) **EP 2 044 910 A1**
(43) Date of publication of application: **08.04.2009**
(21) Application number: 08017290.1
(22) Date of filing: 01.10.2008
(51) Int. Cl.: A61F 7/03

(54) **Heat generating sheet and heat generating sheet bag**

(30) Priority: 04.10.2007 JP 2007260578
(71) Applicant: JAPAN PIONICS CO., LTD., Minato-ku, Tokyo (JP)
(72) Inventor: Ajiri, Asao, Hiratsuka-shi Kanagawa (JP); Matsumoto, Yoshiki, Hiratsuka-shi Kanagawa (JP)
(74) Representative: Leifert & Steffan

(57) **Abstract**

The present invention relates to a heat generating sheet which includes a plurality of flat heat generating elements disposed in a plane, and two packaging sheet materials between which the flat heat generating elements are interposed, wherein the two packaging sheet materials each have a boomerang shape and a perforation line from which each packaging sheet material is divided into halves, and the two packaging sheet materials and the flat heat generating elements interposed therebetween are thermocompression-bonded together; and a heat generating sheet bag which includes the heat generating sheet, and an air-impermeable flat bag in which the heat generating sheet is enclosed in a hermetically sealed condition, and more specifically, relates to a heat generating sheet accommodating heat generating elements capable of generating heat by contact with oxygen in air; and a heat generating sheet bag enclosing the heat generating sheet in a hermetically sealed condition. There are provided a heat generating sheet and a heat generating sheet bag which are applied to flexions such as elbows, knees and neck in a well-fit manner and can be easily used without a strange feel.

## Description

### BACKGROUND OF THE INVENTION

The present invention relates to a heat generating sheet and a heat generating sheet bag, and more particularly to a heat generating sheet which is applied to flexions such as elbows, knees and neck in a well-fit manner without strange feel, and a heat generating sheet bag enclosing the heat generating sheet in a hermetically sealed condition.

Hitherto, there have been used disposable portable body warmers or the like which are constituted from an air-permeable flat bag filled with a heat generating composition containing an oxidizable metal, an activated carbon, an inorganic electrolyte, water, a water-retaining agent, etc., which is capable of generating heat when contacted with oxygen in air, and an air-impermeable outer bag enclosing the air-permeable flat bag therein for preventing the composition from contacting with outside air before used and avoiding diffusion of vaporized water to outside. These portable body warmers have various heat generating characteristics suitably set depending upon portions to which the portable body warmers are applied, and are commercially available in the form of various kinds of products of body-applying type, pocket type and footwear type. Some of the conventional portable body warmers have an adhesive layer to facilitate fitting to the desired portions upon use.

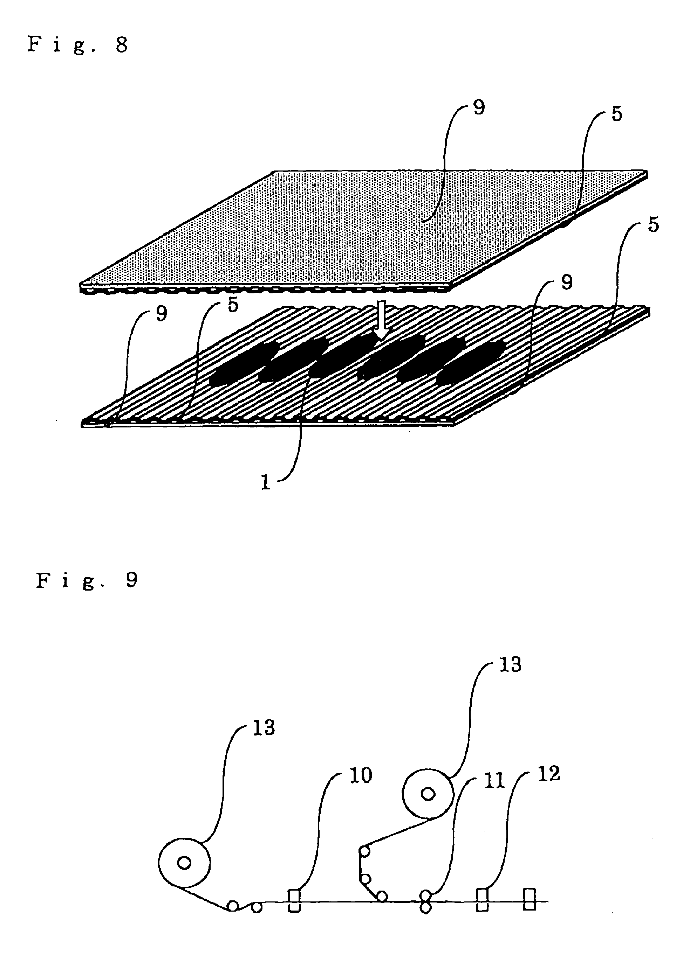

In addition, there have also been used sheet-like disposable portable body warmers in which a heat generating composition is retained in voids of a nonwoven fabric in order to prevent the composition from being locally gathered or concentrated during use.
Such disposable portable body warmers are produced, for example, by the method as shown in Fig. 9 in which a heat generating composition and a heat-fusible resin powder are scattered over a sheet-like nonwoven fabric continuously fed through respective rotating rolls, and then another sheet-like nonwoven fabric is continuously fed and overlapped on the former nonwoven fabric, followed by thermocompression-bonding these overlapped sheets using a mold press to form an integrated laminate, cutting the resultant laminate into a desired size and enclosing the obtained cut elements in an air-impermeable outer bag in a hermetically sealed manner.

Further, in addition to the above portable body warmers, there are also known disposable portable body warmers which have been developed for fitting to flexions such as elbows, knees and neck upon use. For examples, there are known a disposable portable body warmer having a heat generating portion filled with a heat generating composition which is divided into a plurality of sections by a sealed portion, as described in UM 6-26829A, and an stretchable disposable portable body warmer in which a disposable portable body warmer portion is disposed on a desired surface of a stretchable material to well fit the portion to a human body, as described in UM 6-61222A.

The conventional disposable portable body warmer having a heat generating portion filled with a heat generating composition which is divided into a plurality of sections is easily folded along the sealed portion even when the heat generating composition is hardened owing to the reaction with oxygen. However, since the sealed portion has a poor stretchability and the respective sections of the disposable portable body warmer have a rectangular shape, fitting of the portable body warmer to flexions such as elbows, knees and neck tends to be difficult and made only with a strange feel. On the other hand, the conventional disposable portable body warmer in which a disposable portable body warmer portion is disposed on a desired surface of a stretchable material tends to be hardened at its fitted portions as the reaction of the heat generating composition with oxygen proceeds, and the hardened portions tend to become less stretchable, resulting in difficulty in fitting the portable body warmer to flexions such as elbows, knees and neck.

### SUMMARY OF THE INVENTION

Accordingly, an object of the present invention is to solve the above conventional problems, and provide a heat generating sheet and a heat generating sheet bag which are well fitted to flexions such as elbows, knees and neck upon use and can be easily used without a strange feel.

As a result of extensive and intensive researches for solving these conventional problems, the present inventors have found that a heat generating sheet obtained by interposing a plurality of flat heat generating elements between two packaging sheet materials each having a boomerang shape and a perforation line from which each packaging sheet material is divided into halves and then thermocompression-bonding the flat heat generating elements and the two packaging sheet materials together, can be readily fitted to flexions such as elbows, knees and neck, and easily usable without a strange feel. The heat generating sheet and the heat generating sheet bag according to the present invention have been completed on the basis of the above finding.

Thus, the present invention relates to a heat generating sheet including a plurality of flat heat generating elements disposed in a plane, and two packaging sheet materials between which the flat heat generating elements are interposed, wherein the two packaging sheet materials each have a boomerang shape and a perforation line from which each packaging sheet material is divided into halves, and the two packaging sheet materials and the flat heat generating elements interposed therebetween are thermocompression-bonded together.
Also, the present invention relates to a heat generating sheet bag including the above heat generating sheet and an air-impermeable flat bag in which the heat generating sheet is enclosed in a hermetically sealed condition.

The heat generating sheet of the present invention having an outer contour of a boomerang shape can be easily fitted to elbows, knees, neck, etc., by allowing a recessed portion of the boomerang shape to contact with a thick cylindrical portion of the elbows, knees, neck, etc., when directly attached to human body upon use. In addition, with the provision of the perforated line from which the heat generating sheet is divided into halves, the heat generating sheet may be partially or completely cut along the perforation line and, as a result, can be used in such a condition as well fitted to elbows, knees, neck, etc., without a strange feel.

### BRIEF DESCRIPTION OF THE DRAWINGS

Fig. 1 is a plan view showing an example of a heat generating sheet according to the present invention.
Fig. 2 is a sectional view showing an example of a heat generating sheet according to the present invention.
Fig. 3 is a perspective view showing an example of a laminated packaging sheet material used in the heat generating sheet of the present invention (first embodiment).
Fig. 4 is a perspective view showing an example of a laminated packaging sheet material used in the heat generating sheet of the present invention (second embodiment).
Fig. 5 is a perspective view showing an example of a laminated packaging sheet material used in the heat generating sheet of the present invention (third embodiment).
Fig. 6 is a sectional view showing an example of a heat generating sheet using a stretchable laminated packaging sheet material.
Fig. 7 is a perspective view showing an example of the heat generating elements disposed on the laminated packaging sheet material.
Fig. 8 is a perspective view showing an example of the condition in which the laminated packaging sheet materials are disposed on both surfaces of the respective heat generating elements and overlapped on each other such that a heat-fusible surface of each packaging sheet material faces inside.
Fig. 9 is a view showing an example of construction of an apparatus used for production of the heat generating sheet of the present invention.
Fig. 10 is a perspective view showing an example of a heat generating sheet bag according to the present invention (partially cut-away perspective view).

### [Explanation of Reference Numerals]

1: Heat generating element; 2: Perforation line; 3: Packaging sheet material; 4: Recessed portion; 5: Heat-fusible sealing material having a repeating wave shape in section; 6: Heat-fusible sealing material having a repeating irregular shape in section; 7: Through-opening; 8: Heat-fusible sealing material having through-openings; 9: Stretchable base material; 10: Section where the heat generating element is disposed (coated or printed) on the packaging sheet material; 11: Heat-pressing section; 12: Cutting section; 13: Roll on which a laminated packaging sheet material is wound; 14: Heat generating sheet; 15: Air-impermeable flat bag; 16: Air-impermeable packaging material.

### DETAILED DESCRIPTION OF THE PREFERRED EMBODIMENTS

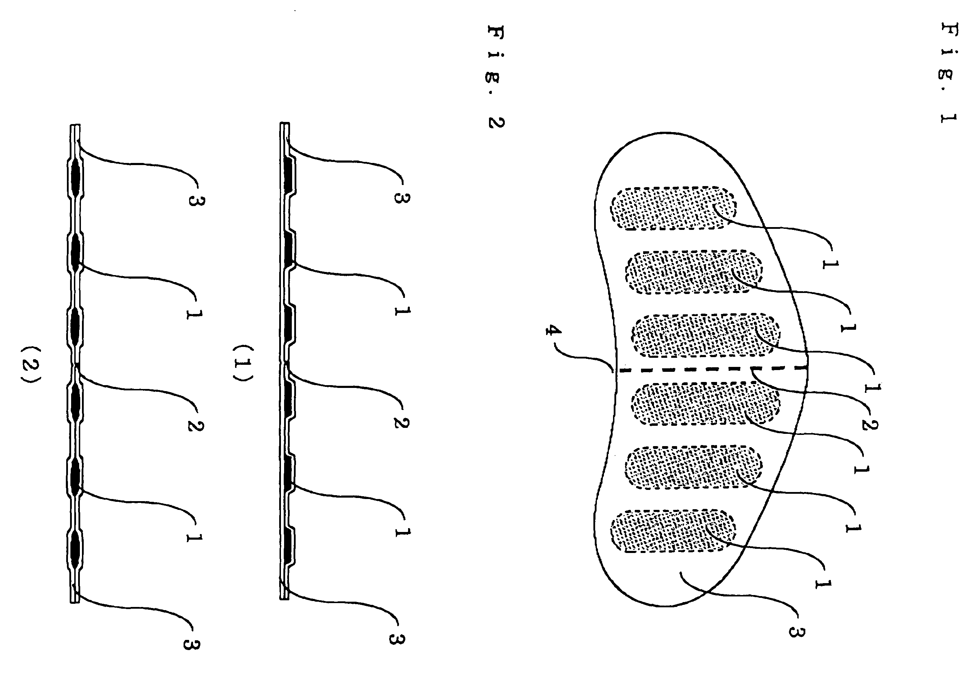

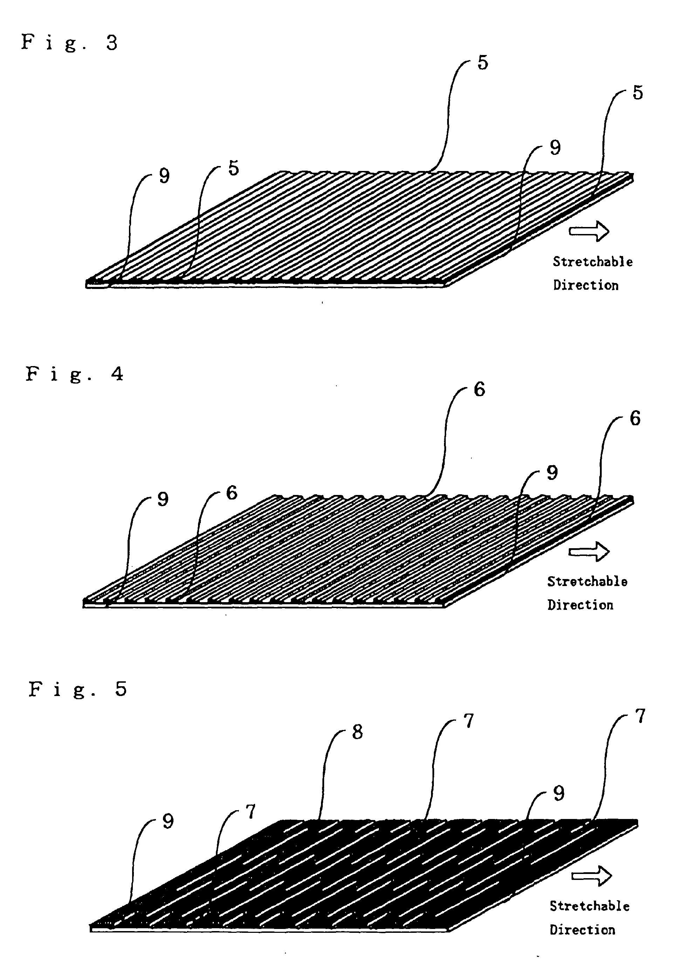

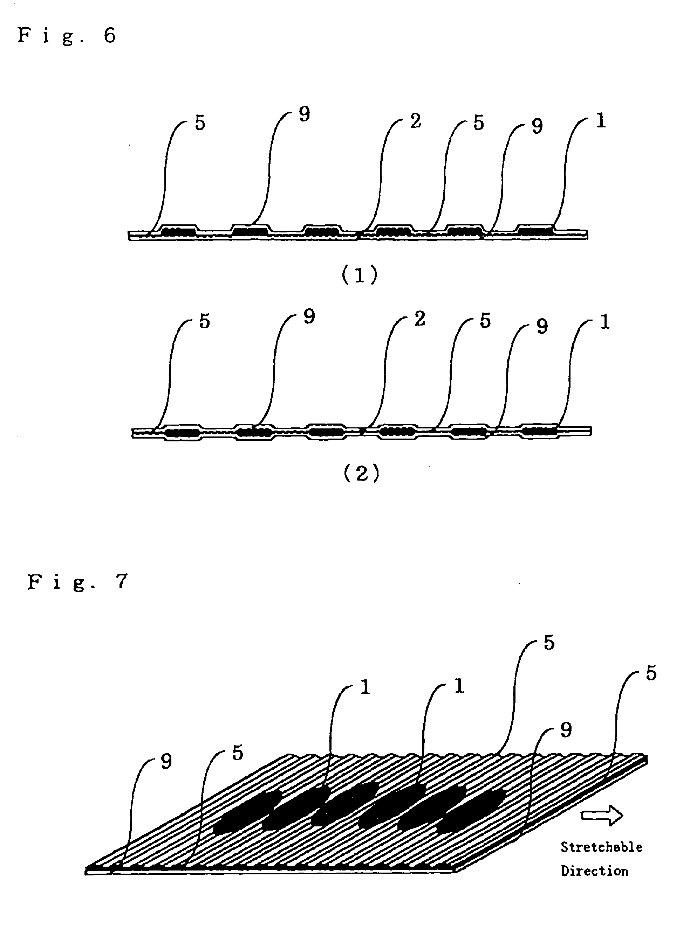

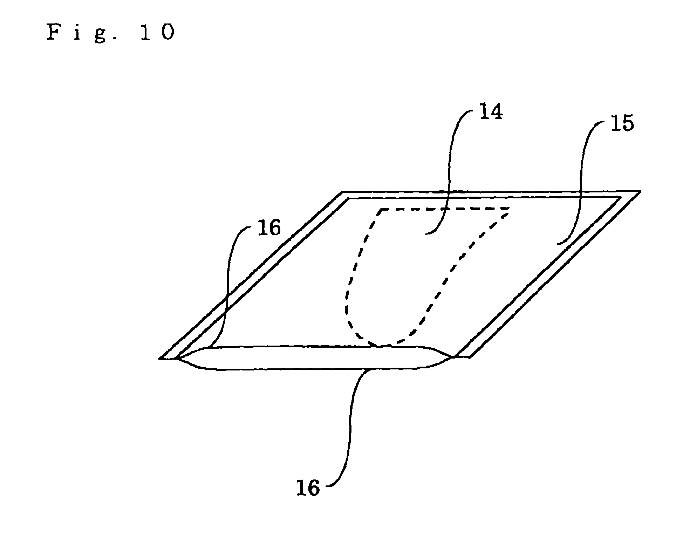

The heat generating sheet and the heat generating sheet bag according to the present invention are applied to disposable portable body warmers for warming, in particular, flexions such as elbows, knees and neck.
In the followings, the heat generating sheet and the heat generating sheet bag according to the present invention are described in detail by referring to Figs. 1 to 10. However, it should be noted that these embodiments are only illustrative and not intended to limit the invention thereto. Meanwhile, Fig. 1 is a plan view showing an example of a heat generating sheet according to the present invention; Fig. 2 is a sectional view showing an example of a heat generating sheet according to the present invention; Figs. 3 to 5 are respectively a perspective view showing an example of a laminated packaging sheet material used in the heat generating sheet of the present invention; Fig. 6 is a sectional view showing an example of a heat generating sheet using a stretchable laminated packaging sheet material; Fig. 7 is a perspective view showing an example of the heat generating elements disposed on the laminated packaging sheet material; Fig. 8 is a perspective view showing an example of the condition
in which the laminated packaging sheet materials are disposed on both surfaces of the heat generating element and overlapped on each other such that a heat-fusible surface of each packaging sheet material faces inside; Fig. 9 is a view showing an example of construction of an apparatus used for production of the heat generating sheet of the present invention; and Fig. 10 is a perspective view showing an example of a heat generating sheet bag according to the present invention (partially cut-away perspective view).

As shown in the plan view of Fig. 1 and the sectional view of Fig. 2, the heat generating sheet of the present invention has such a structure in which a plurality of flat heat generating elements 1 disposed in a plane are interposed between two packaging sheet materials 3 having a boomerang shape and a perforation line from which each packaging sheet material is divided into halves, and is produced by thermocompression-bonding the two packaging sheet materials and the heat generating elements interposed therebetween together.
The heat generating sheet of the present invention is used, for example, in the following manner. That is, the heat generating sheet is first opened by appropriately cutting it along the perforation line, and then a recessed portion 4 of the boomerang shape thereof is attached to a neck of a person from his back side, thereby allowing the heat generating sheet to well fit to the neck. If it is intended to bring a whole surface of the conventional rectangular heat generating sheet having no perforation line into close contact with a region extending from a neck to a shoulder, there tend to occur inconveniences such as application of excessively high force to the heat generating sheet and formation of wrinkles, resulting in occurrence of a strange feel upon use. On the other hand, the heat generating sheet of the present invention is free from such inconveniences.

Meanwhile, the "boomerang shape" of the heat generating sheet of the present invention as used herein means a generally U-character or V-character shape or a curved shape like a parenthesis symbol " ( " which can be divided into halves. In the heat generating sheet of the present invention, the perforation line may be formed only at one portion along a center thereof as shown in Fig. 1, or may be formed at a plurality of portions. For example, the perforation line may be provided at all the portions between adjacent ones of the heat generating elements. In addition, the heat generating sheet of the present invention is usually symmetrical relative to the perforation line as an axis, and the number of the heat generating elements is usually an even number.

The packaging sheet material used in the heat generating sheet of the present invention is usually in the form of a laminated packaging sheet material including a heat-fusible sealing material and a base material. Upon producing the heat generating sheet of the present invention, two sheets of such a laminated packaging sheet material are usually used. The two laminated packaging sheet materials and the heat generating elements are integrally thermocompression-bonded to each other to form such a layer structure composed of a base material/a heat-fusible sealing material/a heat generating element/a heat-fusible sealing material/a base material, and the resultant laminate is cut into a boomerang shape. However, the heat-fusible sealing material generally tends to be hardened upon heating and no longer show a stretchability. Therefore, in order to further enhance a fittability of the heat generating sheet to elbows, knees, neck, etc., it is preferred to selectively use a laminated packaging sheet material whose stretchability can be still maintained after heating.

Examples of the preferred laminated packaging sheet material include a laminated packaging sheet material as shown in Fig. 3 which is composed of a heat-fusible sealing material 5 having a repeating wave shape in section, and a stretchable base material 9 (hereinafter referred to as a "laminated packaging sheet material of a first embodiment"), a laminated packaging sheet material as shown in Fig. 4 which is composed of a heat-fusible sealing material 6 having a repeating irregular shape in section, and a stretchable base material 9 (hereinafter referred to as a "laminated packaging sheet material of a second embodiment"), and a laminated packaging sheet material as shown in Fig. 5 which is composed of a heat-fusible sealing material 8 having a plurality of through-openings 7, and a stretchable base material 9 (hereinafter referred to as a "laminated packaging sheet material of a third embodiment").

The respective laminated packaging sheet materials as described above are obtained by overlapping a heat-fusible sealing material containing a heat-fusible component such as, for example, polyethylene and polypropylene, and a stretchable base material on each other, and then heat-fusing these overlapped materials together using a roll, a metal mold, a mold press, etc., which have a press surface of a wave or irregular shape, or by overlapping the heat-fusible sealing material having a plurality of through-openings and the stretchable base material on each other, and then heat-fusing these overlapped materials together using a roll, a metal mold, a mold press, etc., which have a flat press surface, so as to form a configuration as shown in each of Figs. 3 to 5.

Meanwhile, the heat-fusible sealing material used in the present invention preferably contains not only the heat-fusible component such as polyethylene and polypropylene, but also a rubber component such as butadiene, isoprene, styrene and urethane. Examples of such a heat-fusible sealing material include a heat-fusible sheet obtained by laminating a sheet containing the rubber component and a sheet containing the heat-fusible component, a heat-fusible sheet obtained by allowing a heat-fusible resin powder to adhere onto a surface of a sheet containing the rubber component, and a heat-fusible sheet obtained by mixing the rubber component and the beat-fusible component with each other and subjecting the resultant mixture to melting and stretching.
Also, the stretchable base material used in the present invention is not particularly limited, and may be either a material that is not contracted again once stretched, or a material that is stretchable and retractable. Examples of the stretchable base material usable in the present invention include various woven fabrics and nonwoven fabrics. Among these materials, the nonwoven fabrics are preferred from the viewpoints of an adequate stretchability and a good touch feel.

In the present invention, the laminated packaging sheet material according to the first embodiment ensures a stretchability by expansion and contraction at a wave-shaped portion of the heat-fusible sealing material 5; the laminated packaging sheet material according to the second embodiment ensures a stretchability by expansion and contraction at a concaved portion (thin wall portion) of the heat-fusible sealing material 6; and the laminated packaging sheet material according to the third embodiment ensures a stretchability by expansion and contraction at the through-opening 7 of the heat-fusible sealing material 8. For example, when using the laminated packaging sheet material according to the first embodiment, the heat generating sheet is finally formed into the configuration as shown in Fig. 6. The heat generating sheet shown in Fig. 6 exhibits a stretchability at portions 5 of the heat-fusible sealing material having a repeating wave shape (except for portions where the heat generating elements 1 are disposed). The heat generating sheets using the laminated packaging sheet materials according to the second and third embodiments, respectively, exhibit a stretchability in the same manner as in the first embodiment.

The heat generating sheet of the present invention is preferably produced so as to exhibit a stretchability in a longitudinal direction of the boomerang shape (usually a direction perpendicular to the perforation line). The maximum elongation [(L - L₀)/L₀] of the laminated packaging sheet material in its stretchable direction is usually from 30 to 200% as measured at an ordinary temperature (25°C), and the maximum elongation of the laminated packaging sheet material in its unstretchable direction is usually less than 30% as measured at an ordinary temperature (25°C). The heat generating sheet of the present invention using such a laminated packaging sheet material exhibits a stretchability at the above portions where no heat generating elements are provided, and is therefore further improved in fittability to elbows, knees, neck, etc.

In the laminated packaging sheet material according to the first embodiment of the present invention (Fig. 3), the heat-fusible sealing material 5 has a wave shape in section. Examples of an individual sectional shape forming the wave shape of the heat-fusible sealing material include a semi-circular shape, a semi-elliptical shape, a bow shape, a mountain shape (trapezoidal shape), a U-character shape, a V-character shape, or other similar shape which are capable of forming the wave shape by repeatedly bonding these shapes to each other at their ends while rotating by 180° around each end. The height of the wave shape (distance between the highest and lowest positions) is usually from 0.05 to 2 mm and preferably from 0.1 to 1 mm, and the width of the wave shape (wavelength) is usually from 0.5 to 20 mm and preferably from 1 to 10 mm.

In the laminated packaging sheet material according to the second embodiment of the present invention (Fig. 4), the sectional shape of the convex portion of the heat-fusible sealing material 6 includes, for example, a semi-circular shape, a semi-elliptical shape, a square shape, a rectangular shape, a trapezoidal shape, a triangular shape, and other shapes similar to these shapes. The height of the convex portion relative to the concave portion is usually from 0.02 to 2 mm and preferably from 0.05 to 1 mm, and the width of the convex or concave portion is usually from 0.5 to 20 mm and preferably from 1 to 10 mm. Meanwhile, although the heat-fusible sealing material in the form of a flat sheet having no convex nor concave portion still exhibits a stretchability by reducing its thickness, there tend to occur inconveniences such as breakage of the sealing material upon the thermocompression bonding and peeling of the sealing material from the heat generating elements due to poor bonding strength therebetween. In the present invention, since an apex of the convex portion of the heat-fusible sealing material is thermocompreseion-bonded to the heat generating elements, the above conveniences can be eliminated.

In the laminated packaging sheet material according to the third embodiment of the present invention (Fig. 5), the planar shape of the respective elongated through-openings 7 includes, for example, an elliptical shape, a semi-circular shape, a rectangular shape, a trapezoidal shape, a triangular shape, and other shapes similar to these shapes. The width of the respective elongated through-openings (including merely a slit) is usually 20 mm or less and preferably from 1 to 10 mm. Meanwhile, the laminated packaging sheet material according to the third embodiment is preferably also provided at a periphery thereof with through-openings (notches) to allow an entire portion of the packaging sheet material to exhibit a stretchability.

Upon producing the heat generating sheet of the present invention, as shown in Fig. 7, a plurality of the heat generating elements 1 of a flat shape are disposed on the heat-fusible side surface (heat-fusible sealing material) of the laminated packaging sheet material. The shape of the respective heat generating elements is not particularly limited, and may include, for example, a circular shape, an elliptical shape, a semi-circular shape, a square shape, a rectangular shape, a polygonal shape, a trapezoidal shape, a rhombus shape, a parallelogram shape, a triangular shape, and other shapes similar to these shapes. Since the heat generating elements have no stretchability, the heat generating elements must be disposed so as not to adversely affect the stretchability of the heat generating sheet. For example, the maximum elongation [(L - L₀)/L₀] of the heat generating sheet in one stretchable direction thereof tends to be substantially in proportion to the value obtained by subtracting a length of a portion where the heat generating elements are present, from a portion exhibiting a stretchability.

The heat generating elements used in the present invention are respectively constituted from a heat generating composition capable of generating heat by contacting with oxygen in air, which contains, for example, an oxidizable metal such as iron, an activated carbon, an inorganic electrolyte such as sodium chloride, water and a water-retaining agent. However, it is required that a plurality of the flat heat generating elements are fixed on the surface of the laminated packaging sheet material as shown in Fig. 6. Therefore, the heat generating elements preferably contain, in addition to the above constitutional components, a thickening agent in order to allow the heat generating elements to fixedly adhere onto the surface of the laminated packaging sheet material by a coating or printing method. The amounts of these ordinary constitutional components used in the heat generating elements are adjusted such that the content of the oxidizable metal therein is from 20 to 80% by mass; the content of the activated carbon therein is from 1 to 10% by mass; the content of the inorganic electrolyte therein is from 1 to 10% by mass; the content of water therein is from 5 to 50% by mass; the content of the water-retaining agent therein is from 1 to 10% by mass; and the content of the thickening agent therein is from 0.1 to 10% by mass.

After disposing a plurality of the flat heat generating elements on the surface of the laminated packaging sheet material, as shown in Fig. 8, an additional laminated packaging sheet material is overlapped over the opposite surface of the flat heat generating elements disposed on the above laminated packaging sheet material such that the heat-fusible side surface thereof faces to the heat generating elements, and then the thus obtained structure is subjected to thermocompression bonding to form an integrated product. In this case, the two laminated packaging sheet materials may be different in kinds from each other, and are preferably identical in kinds to each other. Also, the two laminated packaging sheet materials are preferably disposed such that the stretchable directions thereof are aligned with each other.

Meanwhile, in the present invention, at least one of the two laminated packaging sheet materials is in the form of an air-permeable packaging material. In addition, in order to facilitate application of the heat generating sheet onto flexions such as elbows, knees and neck, an adhesive layer may be further provided on the surface of one of the laminated packaging sheet materials, preferably the air-impermeable laminated packaging sheet material, which surface is opposed to the heat-fusible surface. The adhesive layer may be covered with a release paper, etc., during the period before use. After completing the above procedure, the laminated packaging sheet materials between which the heat generating elements are interposed, are cut into a boomerang shape, and then the perforation line is formed thereon, thereby obtaining the heat generating sheet as aimed.

The above heat generating sheet may be produced in the following manner. For example, using a production apparatus having a heat generating element applying (coating or printing) section 10, a thermocompression bonding section 11, a cutting section 12 (for cutting into a boomerang shape), etc., (which is further provided at any position thereof with a device for forming a perforation line) as shown in Fig. 9, a roll 13 of the laminated packaging sheet material (or a laminate formed by laminating an adhesive layer and a release paper on the laminated packaging sheet material) is loaded to the production apparatus, and then the operation of the production apparatus starts to continuously produce the heat generating sheet. In this case, when the stretchable direction of the laminated packaging sheet material is aligned with the feed direction thereof (the direction in which a tensile force is applied), it may be difficult to maintain a constant feed rate of the laminated packaging sheet material owing to its stretchability. Therefore, the non-stretching direction of the laminated packaging sheet material is aligned with the feed direction thereof, thereby enabling production of the heat generating sheet in an efficient and facilitated manner.

The heat generating sheet bag of the present invention is formed, as shown in Fig. 10, by hermetically enclosing the above heat generating sheet 14 as such or in a two-folded state in an air-impermeable flat bag 15. Upon producing the heat generating sheet bag, two air-impermeable sheet materials 16 made, for example, of polyethylene, polypropylene, etc., are overlapped on each other, and after heat-fusing peripheral sides of the overlapped sheet materials except for one peripheral side thereof to form a bag, the heat generating sheet 14 is inserted into the obtained bag. Then, the one peripheral side of the bag which remains opened is heat-fused to thereby produce the heat generating sheet bag.

Next, the present invention is described in more detail by referring to the following examples. However, these examples are only illustrative and not intended to limit the present invention thereto.

### EXAMPLES

### EXAMPLE 1

### (Production of Laminating Packaging Sheet Material)

A heat-fusible sheet (thickness: 70 µm) as a heat-fusible sealing material composed of a commercially available heat-fusible component sheet (low-temperature fusible sheet)/a rubber component sheet/a heat-fusible component sheet (high-temperature fusible sheet), and a commercially available nonwoven fabric (polyester spun lace; thickness; 200 µm) as a stretchable base material were overlapped on each other such that the surface of the heat-fusible component sheet (low-temperature fusible sheet) was disposed inside. The thus overlapped sheets were interposed between two mold plates each having a mold surface of a repeating wave shape extending in one direction thereof, and heat-fused together, thereby obtaining a laminated packaging sheet material composed of the heat-fusible sealing material 5 and the stretchable base material 9 which had a repeating wave shape in section as shown in Fig. 3. Meanwhile, upon the heat-fusing operation, the mold temperature was adjusted to the temperature at which the heat-fusible component sheet (low-temperature fusible sheet) was melted but the heat-fusible component sheet (high-temperature fusible sheet) still remained unmelted. The height of the wave shape of the thus obtained laminated packaging sheet material (distance between the highest and lowest positions) was 0.2 mm, and the width of the wave shape (wavelength) was 6 mm. Further, the laminated packaging sheet material was cut into a size of 100 mm x 220 mm. The two laminated packaging sheet materials cut into the above size were produced, and one of the laminated packaging sheet materials were provided on a surface thereof with needle apertures to render it air-permeable.

### (Production of Heat Generating Sheet and Heat Generating Sheet Bag)

Thirty grams of a coating solution prepared by mixing 63.1% by mass of an iron powder, 3.2% by mass of an activated carbon, 1.9% by mass of a common salt (as an inorganic electrolyte), 26.5% by mass of water, 0.6% by mass of a water-retaining agent and 4.7% by mass of a thickening agent in a nitrogen gas atmosphere, was applied onto separate 6 positions on the heat-fusible side surface of the above laminated packaging sheet material (air-impermeable sheet), thereby obtaining the laminated packaging sheet material on which the heat generating elements were disposed as shown in Fig. 7. Meanwhile, the shape of the respective heat generating elements was an elliptical shape having a major axis diameter of 50 mm and a minor axis diameter of 18 mm, and each element had a thickness of 1.0 mm.

Next, as shown in Fig. 8, the above laminated packaging sheet material (air-permeable sheet material) was overlapped over the surface of the heat generating elements formed on the air-impermeable sheet material such that the heat-fusible side surface thereof faced to the heat generating elements, and the stretchable directions of the two laminated packaging sheet materials were aligned with each other, and the overlapped sheets and the heat generating elements interposed therebetween were integrally thermocompression-bonded to each other, thereby obtaining the heat generating sheet as shown in Fig. 6(1). In addition, in order to apply the heat generating sheet onto flexions such as elbows, knees and neck, an adhesive layer was formed on the surface of the air-impermeable laminated packaging sheet material, and further the thus formed adhesive layer was covered with a release paper.
The thus formed heat generating sheet was cut into a boomerang shape (having a longitudinal length of 210 mm and a depth of its concave portion of 10 mm), and a perforation line (having a length of 85 mm) was formed thereon. The resultant boomerang-shaped heat generating sheet was then two-folded and enclosed in an air-impermeable flat bag having a size of 120 mm x 140 mm in a hermetically sealed manner, thereby producing a heat generating sheet bag.

### (Examination on Stretchability and Fittability of Heat Generating Sheet)

The two heat generating sheet bags were produced by the above method, and allowed to stand in room at 25°C over a whole day and night. The heat generating sheet was taken out of one of the bags, and immediately subjected to measurement of a maximum elongation thereof in its stretchable direction. As a result, it was confirmed that the maximum elongation of the heat generating sheet was 51%.
Next, the heat generating sheet was taken out of the other bag. The release paper was peeled off from the heat generating sheet, and then the heat generating sheet was cut along the perforation line until reaching a half way of the perforation line. Then, the heat generating sheet was applied to a neck of a person from his back side such that a recessed portion of the heat generating sheet was fitted to the neck side. As a result, the heat generating sheet was well fitted to a region from a shoulder to the neck without need of applying an excessively large force and occurrence of wrinkles or without a strange feel. Also, it was confirmed that the maximum temperature of the heat generating sheet upon use was 48°C and the time period for which the heat generating sheet was maintained at a temperature of 40°C or higher was about 5 h. This showed that the heat generating sheet had good heat-generating characteristics.
Further, the heat generating sheet after used (after the temperature was dropped) was subjected to measurement of its maximum elongation in the stretchable direction. As a result, it was confirmed that the maximum elongation was 49% and therefore substantially the same as that before use.

### EXAMPLE 2

### (Production of Heat Generating Sheet and Heat Generating Sheet Bag)

The laminated packaging sheet material composed of a heat-fusible sealing material 6 and a stretchable base material 9 which had a repeating irregular shape as shown in Fig. 4 was produced in the same manner as in Example 1 except that a metal mold having a repeating irregular shape extending in one direction thereof and a flat metal mold were used in place of the two metal molds each having a repeating wave shape extending in one direction thereof as used in Example 1. Meanwhile, in the thus produced laminated packaging sheet material, a thickness of a convex portion thereof was 0.25 mm, a thickness of a concave portion thereof was 0.15 mm, and a width of each of the convex and concave portions was 3 mm. Further, the heat generating sheet was produced in the same manner as in Example 1 except for using the above laminated packaging sheet material, and then enclosed in an air-impermeable flat bag in a hermetically sealed manner to produce a heat generating sheet bag.

### (Examination on Stretchability and Fittability of Heat Generating Sheet)

The two heat generating sheet bags were produced by the above method, and allowed to stand in room at 25°C over a whole day and night. The heat generating sheet was taken out of one of the bags, and immediately subjected to measurement of a maximum elongation thereof in its stretchable direction. As a result, it was confirmed that the maximum elongation of the heat generating sheet was 42%.
Next, the heat generating sheet was taken out of the other bag. The release paper was peeled off from the heat generating sheet, and then the heat generating sheet was cut along the perforation line until reaching a half way of the perforation line. Then, the heat generating sheet was applied to a neck of a person from his back side such that a recessed portion of the heat generating sheet was fitted to the neck side. As a result, the heat generating sheet was well fitted to a region from a shoulder to the neck without need of applying an excessively large force and occurrence of wrinkles or without a strange feel. Also, it was confirmed that the maximum temperature of the heat generating sheet upon use was 47°C and the time period for which the heat generating sheet was maintained at a temperature of 40°C or higher was about 5 h. This showed that the heat generating sheet had good heat-generating characteristics.
Further, the heat generating sheet after used (after the temperature was dropped) was subjected to measurement of its maximum elongation in the stretchable direction. As a result, it was confirmed that the maximum elongation was 40% and therefore substantially the same as that before use.

### EXAMPLE 3

### (Production of Heat Generating Sheet and Heat Generating Sheet Bag)

The laminated packaging sheet material composed of a heat-fusible sealing material 8 having a plurality of elongated through-openings 7 as shown in Fig. 5 and a stretchable base material 9 was produced in the same manner as in Example 1 except that slits each having a width of 1.0 mm were formed at intervals of 5 mm on the heat-fusible sheet, and two flat metal molds were used in place of the two metal molds each having a repeating wave shape. Further, the heat generating sheet was produced in the same manner as in Example 1 except for using the above laminated packaging sheet material, and then enclosed in an air-impermeable flat bag in a hermetically sealed manner to produce a heat generating sheet bag.

### (Examination on Stretchability and Fittability of Heat Generating Sheet)

The two heat generating sheet bags were produced by the above method, and allowed to stand in room at 25°C over a whole day and night. The heat generating sheet was taken out of one of the bags, and immediately subjected to measurement of a maximum elongation thereof in its stretchable direction. As a result, it was confirmed that the maximum elongation of the heat generating sheet was 45%.
Next, the heat generating sheet was taken out of the other bag. The release paper was peeled off from the heat generating sheet, and then the heat generating sheet was cut along the perforation line until reaching a half way of the perforation line. Then, the heat generating sheet was applied to a neck of a person from his back side such that a recessed portion of the heat generating sheet was fitted to the neck side. As a result, the heat generating sheet was well fitted to a region from a shoulder to the neck without need of applying an excessively large force and occurrence of wrinkles or without a strange feel. Also, it was confirmed that the maximum temperature of the heat generating sheet upon use was 48°C and the time period for which the heat generating sheet was maintained at a temperature of 40°C or higher was about 5 h. This showed that the heat generating sheet had good heat-generating characteristics.
Further, the heat generating sheet after used (after the temperature was dropped) was subjected to measurement of its maximum elongation in the stretchable direction. As a result, it was confirmed that the maximum elongation was 42% and therefore substantially the same as that before use.

### COMPARATIVE EXAMPLE 1

### (Production of Heat Generating Sheet and Heat Generating Sheet Bag)

The laminated packaging sheet material composed of a flat heat-fusible sealing material and a stretchable base material was produced in the same manner as in Example 1 except that two flat metal molds were used in place of the two metal molds each having a repeating wave shape. Further, the heat generating sheet was produced in the same manner as in Example 1 except for using the above laminated packaging sheet material without forming the sheet material into a boomerang shape and providing any perforation line thereon. The thus produced heat generating sheet was enclosed in an air-impermeable flat bag in a hermetically sealed manner to produce a heat generating sheet bag.

### (Examination on Stretchability and Fittability of Heat Generating Sheet)

The two heat generating sheet bags were produced by the above method, and allowed to stand in room at 25°C over a whole day and night. The heat generating sheet was taken out of one of the bags, and immediately subjected to measurement of a maximum elongation thereof in its longitudinal direction. As a result, it was confirmed that the maximum elongation of the heat generating sheet was 8%.
Next, the heat generating sheet was taken out of the other bag. After peeling off the release paper from the heat generating sheet, the heat generating sheet was applied to a neck of a person from his back side. As a result, the heat generating sheet suffered from occurrence of wrinkles and a strange feel, and was difficult to use. Whereas, it was confirmed that the maximum temperature of the heat generating sheet upon use was 48°C and the time period for which the heat generating sheet was maintained at a temperature of 40°C or higher was about 5 h, and therefore the heat generating sheet had good heat-generating characteristics.
Further, the heat generating sheet after used (after the temperature was dropped) was subjected to measurement of its maximum elongation in a longitudinal direction thereof. As a result, it was confirmed that the maximum elongation was 4% and therefore further deteriorated as compared to that before use.

As described above, it is apparently recognized that the heat generating sheets obtained in Examples according to the present invention were well fittable to flexions such as neck and easily usable without any strange feel as compared to the heat generating sheet obtained in Comparative Example 1.

## Claims

1. A heat generating sheet comprising a plurality of flat heat generating elements disposed in a plane, and two packaging sheet materials between which the flat heat generating elements are interposed, wherein the two packaging sheet materials each have a boomerang shape and a perforation line from which each packaging sheet material is divided into halves, and the two packaging sheet materials and the flat heat generating elements interposed therebetween are thermocompression-bonded together.

2. The heat generating sheet according to claim 1, wherein the packaging sheet materials are each in the form of a laminated packaging sheet material comprising a heat-fusible sealing material and a stretchable base material.

3. The heat generating sheet according to claim 2, wherein the heat-fusible sealing material has a repeating wave shape or a repeating irregular shape in a cross-section thereof, or a plurality of througb-openings.

4. The heat generating sheet according to claim 2, wherein the stretchable base material is a nonwoven fabric.

5. The heat generating sheet according to claim 1, wherein the flat heat generating elements each have an elongated shape disposed in parallel with the perforation line.

6. The heat generating sheet according to claim 1, wherein the respective flat heat generating elements contain an oxidizable metal, an activated carbon, an inorganic electrolyte, water, a thickening agent and a polymeric water-retaining agent.

7. The heat generating sheet according to claim 1, wherein at least one of the packaging sheet materials is an air-permeable packaging material.

8. The heat generating sheet according to claim 1, further comprising an adhesive layer formed on a surface of one of the packaging sheet materials which surface is opposed to its surface on which the flat heat generating elements are disposed.

9. A heat generating sheet bag comprising the heat generating sheet as defined in claim 1, and an air-impermeable flat bag in which the heat generating sheet is enclosed in a hermetically sealed condition.
